# EUROPEAN PATENT APPLICATION

(11) **EP 1 452 856 A1**
(43) Date of publication of application: **01.09.2004**
(21) Application number: 02786000.6
(22) Date of filing: 02.12.2002
(51) Int. Cl.: G01N 27/327

(54) **BIOSENSOR**

(30) Priority: 05.12.2001 JP 2001371058
(71) Applicant: Matsushita Electric Industrial Co., Ltd., Kadoma-shi, Osaka 571-8501 (JP)
(72) Inventor: Yamamoto, Tomohiro, Hirakata-shi, Osaka 573-0018 (JP); Hasegawa, Miwa, Ako-gun Hyogo 678-1205 (JP); Miyamoto, Yoshiko, Suita-shi, Osaka 565-0821 (JP); Yoshioka, Toshihiko, Hirakata-shi, Osaka 573-0035 (JP)
(74) Representative: Bradley, Josephine Mary
(86) International application number: PCT/JP2002/012607
(87) International publication number: WO 2003/048756

(57) **Abstract**

A biosensor in accordance with the present invention comprises: an electrically insulating base plate; an electrode system comprising at least a measuring electrode and a counter electrode formed on the base plate; a cover member which is joined to the base plate to form a sample solution supply pathway for supplying a sample solution to the electrode system between the cover member and the base plate; and a reaction reagent carried at a position which is exposed to the sample solution supply pathway. The cover member comprises a cover which is disposed at a position opposite to the electrode system formed on the base plate and a spacer which is inserted between the cover and the base plate, the spacer having a slit for forming the sample solution supply pathway, the cover has an air vent communicating with the slit of the spacer, and a part of the cover facing the air vent and constituting the ceiling of the sample solution supply pathway protrudes at its center toward the air vent relative to the side walls of the sample solution supply pathway.

## Description

### Technical Field

The present invention relates to a biosensor for carrying out prompt and highly accurate quantification of a measuring subject in a sample with ease.

### Background Art

Conventionally, the following biosensor has been proposed as a system for simplified quantification of a specific component in a sample without diluting or stirring the sample solution (Japanese Laid-Open Patent Publication Hei No. 2-062952).

This biosensor is produced by forming an electrode system comprising a measuring electrode, a counter electrode and a reference electrode on an electrically insulating base plate by a method such as screen printing and forming a reaction reagent layer comprising a hydrophilic polymer, an oxidoreductase and an electron mediator on the electrode system. A buffer is added to the reaction reagent layer, when necessary.

In order to introduce a sample solution to the vicinity of the electrode system and dissolve the reaction reagent layer to initiate an enzyme reaction, the biosensor includes a cover member which is joined to the base plate to form a sample solution supply pathway for supplying the sample solution to the electrode system between the cover member and the base plate, and the cover member has an air vent for facilitating the introduction of the sample solution. This structure allows the sample solution to be introduced into the sample solution supply pathway by capillarity to reach the vicinity of the electrode system.

When a sample solution containing a substrate is introduced into a biosensor having such a structure, dissolution of the reaction reagent layer takes place, causing a reaction between the enzyme and the substrate, and this enzyme reaction is accompanied by reduction of the electron mediator. Upon completion of the enzyme reaction, the reduced electron mediator is electrochemically oxidized, and based on the oxidation current value upon the oxidation, the concentration of the substrate in the sample solution can be determined.

This biosensor is theoretically applicable to measurements of various substances by using an enzyme whose substrate is the substance to be measured.

For example, the use of glucose oxidase as the oxidoreductase can yield a biosensor for measuring glucose concentration in blood. Glucose sensors based on the above-described principle have found extensive commercial application.

Also, the use of cholesterol oxidase can yield a biosensor for measuring serum cholesterol. However, serum cholesterol level serving as diagnostic standard is the sum of cholesterol and cholesterol ester concentrations.

Since cholesterol ester cannot serve as a substrate of the oxidation reaction by cholesterol oxidase, a process of converting cholesterol ester into cholesterol is necessary for measuring serum cholesterol level as diagnostic standard. Cholesterol esterase is known as an enzyme for catalyzing this process.

Therefore, it is theoretically possible to measure the total cholesterol concentration in serum by a biosensor having a reaction reagent system containing cholesterol esterase and cholesterol oxidase.

However, in such a biosensor having the above-described constitution, when the sample solution is a low-viscosity solution which does not contain protein or the like, the sample solution permeates the sample solution supply pathway so quickly that it may not only fill the sample solution supply pathway but also overflow into the air vent. In this case, the concentration of the dissolved reaction reagent layer somewhat decreases. The concentration of the reduced electron mediator produced by the enzyme reaction depends on the concentration of the substrate in the sample solution regardless of the concentration of the reaction reagents, and it is therefore thought that there is no influence on the response value if a sufficient reaction time is given. However, the decrease in the concentration of the reaction reagent layer may slow the enzyme reaction, possibly resulting in a decrease in the response value.

Also, the reaction reagent layer is generally formed by dropping a mixed aqueous solution containing a hydrophilic polymer, an oxidoreductase and an electron mediator onto the electrode system and drying it. If such a reaction reagent layer contains excessively large amounts of reagents, the reaction reagent layer does not dissolve promptly upon dropping of the sample solution, thereby causing a problem of requiring long time for making a measurement.

Particularly, when the above-described constitution of the biosensor is applied to a cholesterol sensor, the dissolution of the reaction reagent layer requires considerably long time in a measurement, because the use of two kinds of enzymes, cholesterol oxidase and cholesterol esterase, is necessary.

When the reaction reagent layer is formed, partly or wholly, by freeze drying an aqueous solution of reagents, the solubility of the reaction reagent layer can be improved, so that shortening the measuring time is possible (U.S. Pat. No. 6,451,372, for example).

However, in the case of forming, by freeze drying, the reaction reagent layer in the cavity constituting the sample solution supply pathway of the cover member, if the air vent adjacent to the cavity has a round shape, the sample solution supply pathway has such a shape that the side walls thereof protrude toward the air vent relative to the center thereof. Thus, when the reaction reagent solution is dropped in the cavity of the cover member, the surface shape of the contacting part of the reaction reagent solution with the air vent becomes distorted. In the case of drying by freeze drying, the surface shape of the dried reaction reagent layer also becomes distorted, since the solution is dried while the outer shape in a state of solution is almost retained.

In a biosensor having a reaction layer formed in such a manner, the homogeneous solubility of the reaction reagent layer upon the supply of the sample solution is somewhat impaired. Since the thickness of the reaction reagent layer tends to increase particularly near the interface between the reaction reagent layer and the air vent, only the thickened part may remain undissolved. This tendency is particularly remarkable when a whole blood sample is measured by a sensor having a hemocyte-filtering filter in front of the sample solution supply pathway, because the permeation of the filtered liquid (plasma) into the sample solution supply pathway is relatively slow.

In view of the above problems, an object of the present invention is to provide a biosensor enabling accurate measurements.

Another object of the present invention is to provide a biosensor which allows prompt and homogeneous dissolution of reagents necessary for measurements such as an enzyme and an electron mediator into a sample solution and which is therefore capable of prompt and accurate measurements.

### Disclosure of Invention

The present invention provides a biosensor comprising: an electrically insulating base plate; an electrode system comprising at least a measuring electrode and a counter electrode formed on the base plate; a cover member which is joined to the base plate to form a sample solution supply pathway for supplying a sample solution to the electrode system between the cover member and the base plate; and a reaction reagent carried at a position which is exposed to the sample solution supply pathway, wherein the cover member comprises a cover which is disposed at a position opposite to the electrode system formed on the base plate and a spacer which is inserted between the cover and the base plate, the spacer having a slit for forming the sample solution supply pathway, the cover has an air vent communicating with the slit of the spacer, and a part of the cover facing the air vent and constituting the ceiling of the sample solution supply pathway protrudes at its center toward the air vent relative to the side walls of the sample solution supply pathway.

It is preferable that the width of the opening of the air vent in the longitudinal direction of the sample solution supply pathway be 0.5 mm at the maximum and 0.1 mm at the minimum.

It is preferable that at least a part of the reaction reagent be carried on a surface of the cover member exposed to the sample solution supply pathway.

It is preferable that the reaction reagent comprise at least cholesterol oxidase and an enzyme capable of hydrolyzing cholesterol ester.

### Brief Description of Drawings

FIG. 1 is an exploded perspective view of a biosensor in one example of the present invention.
FIG. 2 is a longitudinal sectional view of the biosensor.
FIG. 3 is a plane view of a cover member of the biosensor.
FIG. 4 is a plane view of a cover member of a biosensor in another example of the present invention.
FIG. 5 is a longitudinal sectional view of the cover member of the biosensor with a reagent layer formed on the cover member.
FIG. 6 is a plane view of a cover member of a biosensor in another example of the present invention.
FIG. 7 is a plane view of a cover member of a biosensor in still another example of the present invention.
FIG. 8 is a plane view of a cover member of a biosensor in still another example of the present invention.
FIG. 9 is a longitudinal sectional view of a biosensor in another example of the present invention.
FIG. 10 is a plane view of a cover member of a conventional biosensor.
FIG. 11 is a longitudinal sectional view of the cover member of the biosensor with a reagent layer formed on the cover member.

### Best Mode for Carrying Out the Invention

The present invention relates to improvements in a biosensor comprising: an electrically insulating base plate; an electrode system comprising at least a measuring electrode and a counter electrode formed on the base plate; a cover member which is joined to the base plate to form a sample solution supply pathway for supplying a sample solution to the electrode system between the cover member and the base plate; and a reaction reagent carried at a position which is exposed to the sample solution supply pathway of the base plate and/or the cover member. Specifically, the cover member comprises a cover which is disposed at a position opposite to the electrode system formed on the base plate and a spacer which is inserted between the cover and the base plate, the spacer having a slit for forming the sample solution supply pathway, the cover has an air vent communicating with the slit of the spacer, and a part of the cover facing the air vent and constituting the ceiling of the sample solution supply pathway protrudes at its center toward the air vent relative to the side walls of the sample solution supply pathway.

The present invention makes it possible to keep the amount of the sample solution introduced into the sample solution supply pathway constant, and therefore, keep the concentration of the reagent dissolved in the sample solution constant, so that variations in response value can be eliminated.

In a preferable mode of the present invention, at least a part of the reaction reagent is carried on a surface of the cover member exposed to the sample solution supply pathway. This structure speeds up the dissolution of the reagent particularly in a sensor requiring large amounts of the reagent, enabling prompt measurements.

Embodiments of the present invention will be described with reference to drawings.

### Embodiment 1

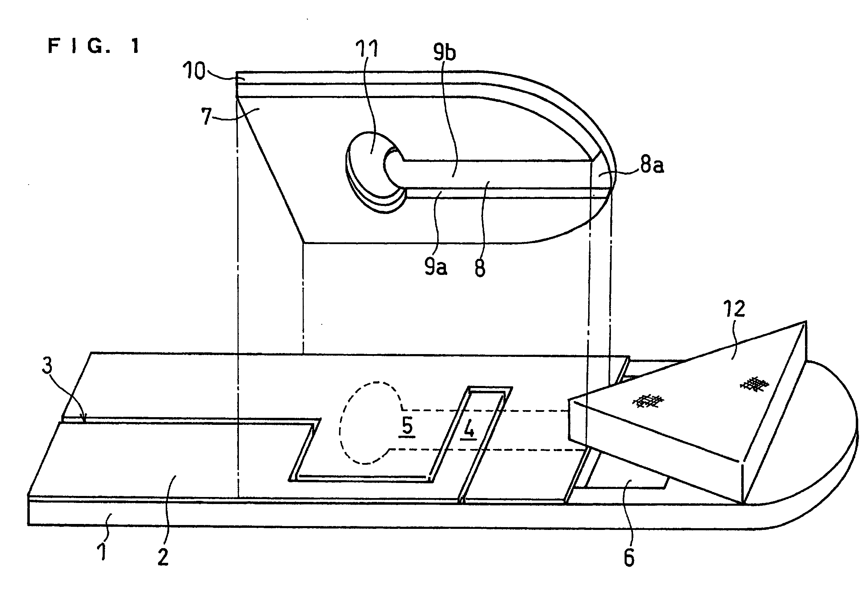

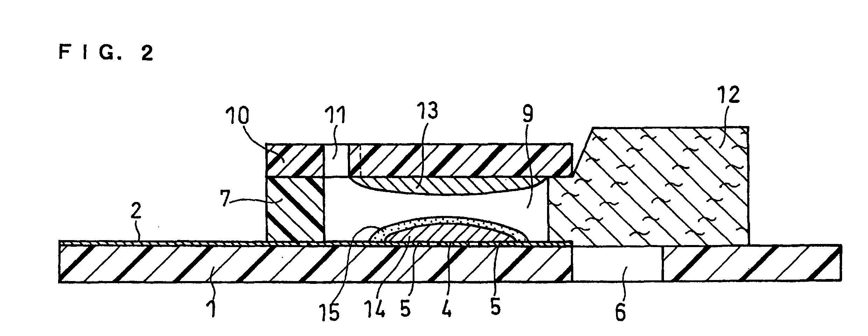

FIG. 1 is an exploded perspective view of a biosensor in this embodiment, and FIG. 2 is a longitudinal sectional view thereof. In FIG. 1, the reaction reagent layer is omitted.

Numeral 1 represents an insulating base plate made of polyethylene terephthalate. A palladium thin film 2 is formed at a specific area of the surface of the base plate 1 by vapor deposition. The thin film 2 is divided into two regions by laser trimming. Numeral 3 represents a portion from which the thin film is removed. One of the two divided regions of the thin film functions as a working electrode 4 and its lead, while the other functions as a counter electrode 5 and its lead. The areas of the electrodes are regulated by the shape of this pattern and by bonding a spacer for forming a sample solution supply pathway that will be described later to the base plate 1. A filter 12 for filtering hemocyte is disposed on the base plate 1 in preparation for the case where the sample solution is blood. A hole 6 is formed in the base plate 1 in order to prevent blood from reaching the sample solution supply pathway from the side faces of the filter 12.

A cover member consisting of the laminate of a spacer 7 and a cover 10 is bonded to the base plate 1 having an electrode system thus formed in the positional relationship as shown by the dash-dotted lines in FIG. 1, to produce a biosensor. The spacer 7 has a slit 8, and the cover 10 has an air vent 11. In this biosensor, a space 9 constituting the sample solution supply pathway is formed in the slit 8 of the spacer 7 between the base plate 1 and the cover 10. An open end 8a of the slit 8 serves as an opening of the sample solution supply pathway, i.e., a sample solution supply inlet. A tip end of the filter 12 is inserted into the sample solution supply inlet. A sample solution dropped to the filter 12 is filtered by the filter 12 and introduced to the electrode system through the sample solution supply pathway. The air vent 11 is so positioned that the distance from the sample supply inlet to the air vent is longer than the distance from the sample supply inlet to the electrode system.

FIG. 2 is a longitudinal sectional view of the above-described biosensor. An enzyme layer 13 is formed on the backside of the cover member forming the sample solution supply pathway. A layer 14 of a sodium salt of carboxymethyl cellulose (hereinafter referred to as CMC), which is a hydrophilic polymer, and a layer 15 of potassium ferricyanide, which is an electron mediator, are formed at positions exposed to the sample solution supply pathway so as to cover the electrode system consisting of the measuring electrode 4 and the counter electrode 5.

The enzyme layer 13, the CMC layer 14 and the potassium ferricyanide layer 15 are formed before the cover member is bonded to the base plate. Specifically, the enzyme layer 13 is formed by laminating the spacer 7 and the cover 10 to fabricate the cover member, placing the cover member in such a manner that the spacer faces upward, dropping an aqueous enzyme solution in the slit 8, and freeze drying it. Also, the layer 14 is formed by dropping an aqueous solution of CMC at an intended position of the base plate 1 for the reagent layer and drying it. Subsequently, the layer 15 is formed by dropping an aqueous solution of potassium ferricyanide over the layer 14 and drying it.

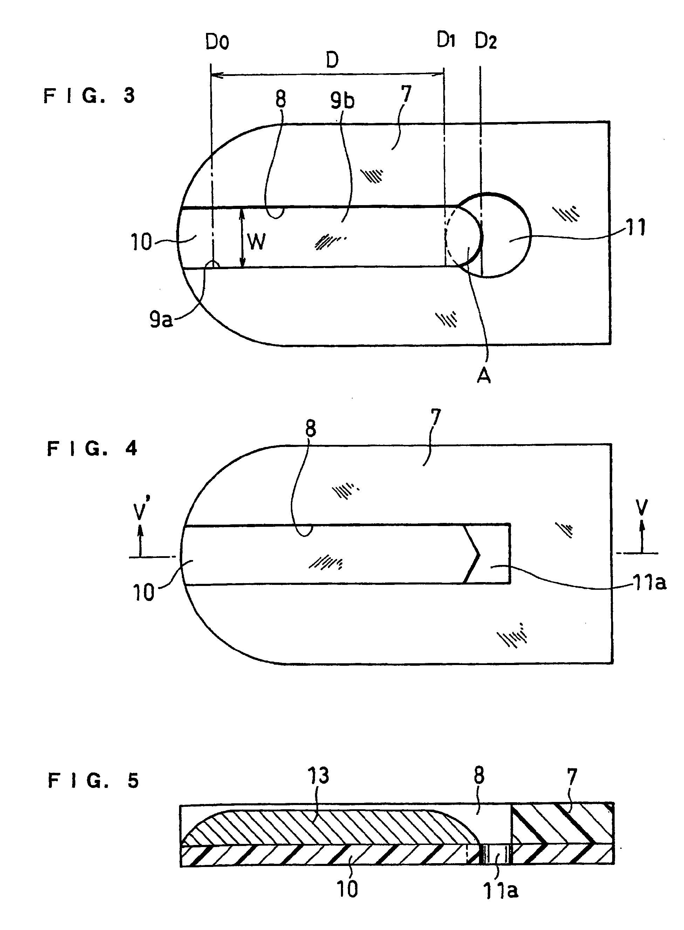

FIG. 3 is a plane view of the cover member in this embodiment with the spacer 7 placed upward. In the sensor comprising the combination of the cover member and the base plate 1, the sample solution supply pathway 9 is formed in the slit 8, and at the end of the sample solution supply pathway 9 is a cavity communicating with the air vent 11. And, a ceiling 9b of the sample solution supply pathway of the cover member 10 has a part facing the air vent 11, and this part protrudes at its center toward the air vent 11 relative to side walls 9a of the sample solution supply pathway. More specifically, in forming the air vent 11 having a diameter greater than the width of the sample solution supply pathway at the end of the liner sample solution supply pathway, the cut-away portion for forming the air vent 11 is regulated such that this part of the ceiling 9b protrudes at its center toward the air vent 11. Thus, when the aqueous reagent solution is dropped in the slit 8 of the cover member, the aqueous solution spreads in such a shape as to protrude toward the air vent at the center of the protruding part of the ceiling.

FIG. 4 illustrates a variation of the cover member. In this example, the width of an air vent 11a is the same as that of the slit 8. The ceiling of the sample solution supply pathway has a part facing the air vent 11a, and this part protrudes at its center, forming a triangle. FIG. 5 is a sectional view of the cover member with the reagent layer cut along line V-V' of FIG. 4.

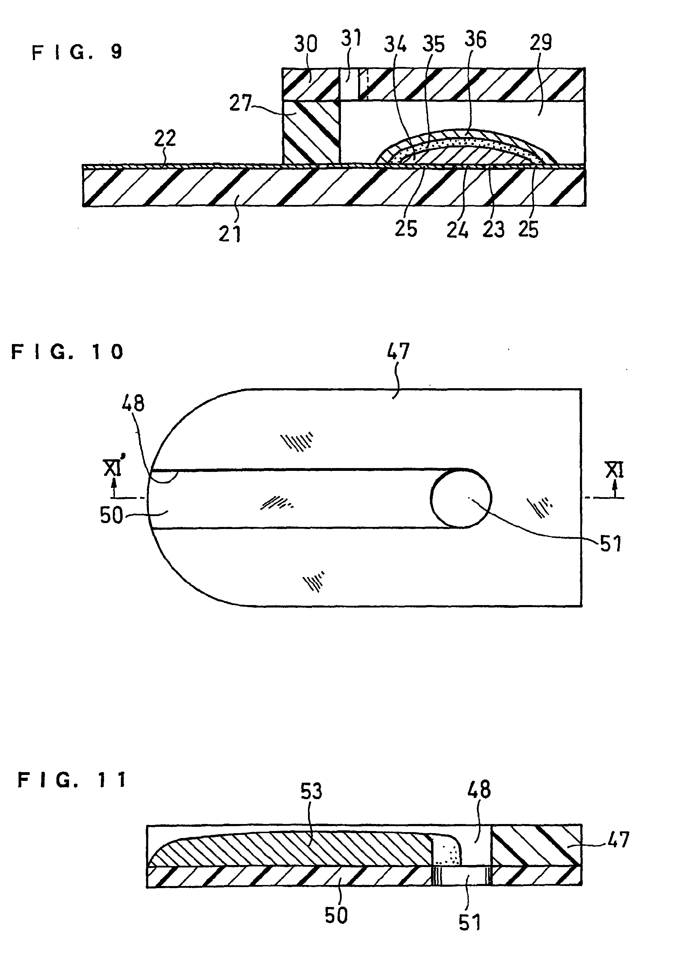

FIG. 10 illustrates a conventional cover member. A slit 48 of a spacer 47 forms a sample solution supply pathway, and an air vent 51 at the end thereof has the same diameter as the width of the slit 48. Thus, the side walls of the sample solution supply pathway and both sides of the ceiling thereof protrude toward the air vent relative to the center of the ceiling. In this structure, an aqueous reagent solution dropped in the slit spreads in such a shape as to protrude toward the air vent along the side walls of the sample solution supply pathway, and when it is freeze dried, it forms a reagent layer 53 while retaining its shape, as illustrated in FIG. 11.

This will be described more specifically. In a structure as illustrated in FIG. 10, the contacting part of the enzyme layer 53 with the air vent 51 is formed when the spread of an enzyme solution dropped for forming the enzyme layer in the sample solution supply pathway is stopped by the air vent. At the interface between the ceiling of the sample solution supply pathway and the air vent, the central part of the ceiling is depressed relative to the parts contacting the right and left side walls of the sample solution supply pathway; thus, at this part, the enzyme solution is stopped and the thickness of the liquid droplet becomes equivalent to that at the right and left side walls or slightly greater than that depending on the surface tension. As a result, the thickness of the contacting part of the formed enzyme layer with the air vent is greater at its center than at the other parts. If the enzyme layer is formed in such a shape, the dissolution of the enzyme layer into the sample solution becomes slow, or some part of the enzyme layer remains undissolved. For example, as in the case of measuring blood by the sensor having the hemocyte-filtering filter as illustrated in FIG. 1, when the permeation of the sample solution (plasma in this case) into the sample solution supply pathway is relatively slow, the enzyme layer having such a shape may cause measuring errors, because the dissolution of the central part of the enzyme layer facing the air vent becomes slow or an undissolved part remains.

On the other hand, in the sensor of this embodiment, the reagent layer 13 is formed in such a shape as to protrude at its center toward the air vent on the ceiling of the sample solution supply pathway, and hence the reagent layer is dissolved smoothly without leaving an undissolved part upon the introduction of the sample solution into the sample solution supply pathway. This enables prompt and accurate measurements.

Also, in the sensor of this embodiment, since the ceiling of the sample solution supply pathway has a part protruding at its center toward the air vent, the amount of the sample solution overflowing into the air vent becomes uniform. Therefore, the amount of the sample solution filled in the sample solution supply pathway becomes uniform, so that the concentration of the reagents dissolved in the sample solution becomes uniform.

Contrary to this, in the conventional structure, the amount of the sample solution filled in the sample solution supply pathway may not become uniform. This will be detailed with reference to FIG. 3. In the conventional sensor, the air vent 11 has a round shape, i.e., the part of the ceiling of the sample solution supply pathway designated by "A" is cut away by the dotted line. In this sensor, the sample solution introduced into the sample solution supply pathway may, depending on the difference in viscosity or the like, stop at the part designated by the dash-dotted line D1 in some cases or overflow up to the point designated by the dash-dotted line D2 in other cases, and hence it is not uniform. For example, in a sensor having a sample solution supply pathway of the dimensions as described in Example 1, the amount of the sample solution filed in the portion from the tip end position D0 of the filter 12 to D2 is larger by approximately 10% than the amount of the sample solution filled in the portion from D0 to D1. Then, the concentration of the reagents dissolved in the sample solution becomes lower accordingly. In this way, in the conventional sensor, the amount of the sample solution filled in the sample solution supply pathway is not uniform. Therefore, the concentration of the reagents in the sample solution is not uniform, and in the case of low reagent concentrations, there are disadvantages of slowed enzyme reaction and lowered response value. Since the sensor of this embodiment is so configured that the ceiling of the sample solution supply pathway has the protruding part as designated by "A" of FIG. 3, the sample solution is invariably filled up to the fixed position as designated by D2. Therefore, the concentration of the reagents in the sample solution becomes uniform, and the response value obtained becomes uniform.

An example of the sample solution having varied viscosities is blood, and its viscosity varies significantly depending on hemocyte inclusion rate (hematocrit value). Even in the case of blood of healthy adults, the hemotocrit value differs among individuals, and even in the same individual, it varies or fluctuates depending on the state of health; it ranges from approximately 30 to approximately 50.

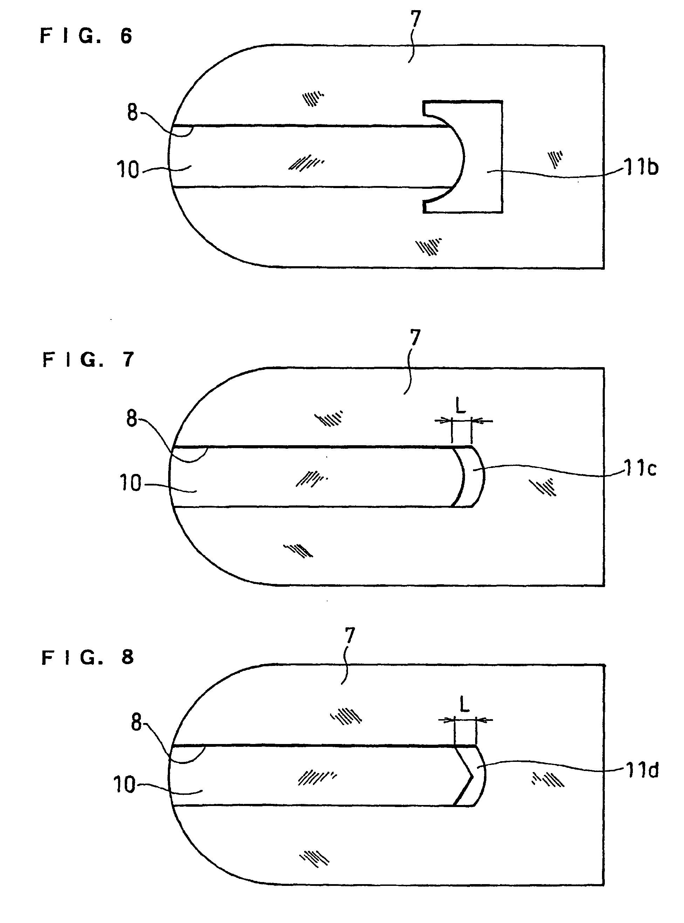

FIG. 6 illustrates another variation of the cover member. In this example, an air vent 11b has a width greater than the slit 8. The ceiling of the sample solution supply pathway has a part protruding toward the air vent, and this part is arc-shaped. In this way, the protruding part of the ceiling of the sample solution supply pathway toward the air vent may be arc-shaped or angular.

In this embodiment, the reaction reagent layers are formed on separate members, i.e., on the cover member and on the base plate. Particularly, a biosensor for measuring the total cholesterol concentration in plasma comprises cholesterol oxidase and cholesterol esterase as enzymes, and preferably comprises a surfactant for activating cholesterol esterase. The biosensor also comprises an electron mediator, a hydrophilic polymer for covering the electrode system, and the like. Therefore, by providing these reagents on two divided members, the dissolution of these reagents into the sample solution can be facilitated. Of the above-listed reagents, it is effective to carry cholesterol oxidase, cholesterol esterase and the surfactant on the cover member and the other reagents on the base plate. Further, a filter for filtering hemocyte in measuring blood is disposed so as to come in contact with the opening of the sample solution supply pathway.

### Embodiment 2

The biosensor in this embodiment has a similar shape to that of FIG. 2. However, with respect to the air vent, the width L of the opening of an air vent 11c in the longitudinal direction of the sample solution supply pathway is 0.5 mm at the maximum and 0.1 mm at the minimum, as illustrated in FIG. 7. In this example, the ceiling of the sample solution supply pathway has a part facing the air vent, and this part is an arc-shaped with its center protruding toward the air vent. And, the width L of the air vent is almost uniform from one side wall, through the center, to the other side wall.

FIG. 8 illustrates a variation of the cover member. The ceiling of the sample solution supply pathway has a part facing the air vent, and this part protrudes at its center toward the air vent, forming a triangle, but the opposing side is arc-shaped. Thus, the width L of an air vent 11d is such that the center is narrower than the parts contacting the side walls of the sample solution supply pathway.

In this embodiment, in the same manner as in Embodiment 1, the ceiling 9b of the sample solution supply pathway of the cover member 10 has a part facing the air vent 11c or 11d, and this part protrudes at its center toward the air vent relative to the side walls 9a of the sample solution supply pathway. This structure produces the same effects as those of Embodiment 1. Further, since the area of the opening of the air vent is regulated in this embodiment, the amount of the sample solution overflowing into the air vent is regulated, thereby making it possible to further suppress the slowdown in the enzyme reaction and the decline in the response value caused by the decrease in the concentration of the reagents in the sample solution.

When a low-viscosity liquid such as water or an aqueous solution of an inorganic salt is used as the sample solution, the permeation of the sample solution into the sample solution supply pathway is so quick that the sample solution may overflow into the air vent without stopping at the interface between the sample solution supply pathway and the air vent. In this case, if the opening of the air vent is wide, the concentration of the reaction reagents decreases due to the permeation of the sample solution into the air vent, so that the enzyme reaction may be slowed, resulting in a decline in the response value. In this embodiment, however, since the area of the opening is reduced to a minimum size by regulating the width of the air vent in the longitudinal direction of the sample solution supply pathway, the decline in the concentration of the reaction reagent layer can be further suppressed.

### Embodiment 3

This embodiment is the same as Embodiment 2 except that all the reaction reagent layers are formed on the base plate. Specifically, the area of the opening of the air vent is regulated. This can suppress the slowdown of the enzyme reaction and the decline in the response value caused by the decrease in the concentration of the reagents in the sample solution.

The biosensor of this embodiment is illustrated in FIG. 9. A palladium thin film 22 is formed on an insulating base plate 21 by vapor deposition, and the thin film 22 is divided into two regions by laser trimming. Numeral 23 represents a portion from which the thin film is removed. One of the two divided regions of the thin film functions as a working electrode 24 and its lead, while the other functions as a counter electrode 25 and its lead.

Over the electrode system on the base plate are formed a CMC layer 34, a layer 35 of an enzyme and an electron mediator, and a layer 36 of lecithin which is an amphipathic lipid. The layers 34 and 35 are formed by dropping an aqueous solution of a reagent or reagents and drying it, and the layer 36 is formed by dropping a solution of lecithin dissolved in an organic solvent, such as a butanol solution, and drying it. The lecithin layer 36 serves to facilitate the introduction of the sample solution and does not take part in the enzyme reaction. A cover member to be joined to the above-described base plate consists of a spacer 27 having a slit for forming a sample solution supply pathway 29 and a cover 30 having an air vent 31. The cover member has a structure as illustrated in FIG. 7.

In the biosensors of the foregoing embodiments, examples of the hydrophilic polymer constituting the hydrophilic polymer layer formed on the electrode system include carboxymethyl cellulose, polyvinyl pyrrolidone, polyvinyl alcohol, ethyl cellulose, hydroxypropyl cellulose, gelatin, polyacrylic acid and its salts, starch and its derivatives, a polymer of maleic anhydride or its salts, polyacrylamide, methacrylate resin, and poly-2-hydroxyethyl methacrylate.

As the electron mediator, ferricyanide ions, p-benzoquinone, phenazine methosulfate, ferrocene and the like may be used. Also, other soluble compounds capable of mediating the electron transfer between the enzyme and the electrode may be arbitrarily used.

As the surfactant, any arbitrary substances having the effect of improving the activity of the enzyme used in the reaction system may be used, and such examples include n-octyl-β-D-thioglucoside, polyethylene glycol monododecyl ether, sodium cholate, dodecyl-β-maltoside, sucrose monolaurate, sodium deoxycholate, sodium taurodeoxycholate, N,N-bis(3-D-gluconamidepropyl)cholamide, N,N-bis(3-D-gluconamidepropyl)deoxycholamide, and polyoxyethylene-p-t-octyl phenyl ether.

As for the method of measuring the oxidation current, a two-electrode system consisting only of a measuring electrode and a counter electrode and a three-electrode system including an additional reference electrode are available, and the three-electrode system enables more accurate measurements.

As to the electrode base plate, the following examples employ a base plate comprising a palladium-deposited base plate with a pattern formed by a laser trimming device, but it is also possible to use a base plate produced by forming silver electrodes on an insulating base plate using a silver paste or the like and coating them with a carbon paste. In this case, since the surfactant may come in direct contact with the silver lead thorough the coating of the carbon paste, precautionary measures may be necessary in designing the shape of the pattern of the silver lead.

In the following, the present invention will be detailed, citing specific examples.

### Example 1

The biosensor of this example has the structure as illustrated in FIGs. 1 and 2.

First, a 0.5 wt% aqueous solution of CMC was dropped in an amount of 5 µl over the electrode system on the base plate 1 and was dried by a hot dryer at 50°C for 20 minutes to form the CMC layer 14. An aqueous solution containing 70 millimole/l potassium ferricyanide, 20 millimole/l sodium cholate and 0.1 wt% CMC was dropped in an amount of 4 µl over the CMC layer 14 and was dried by a hot drier at 50°C for 10 minutes to form the potassium ferricyanide layer 15.

Meanwhile, a mixed solution was prepared by dissolving cholesterol oxidase (hereinafter referred to as ChOD), cholesterol esterase (hereinafter referred to as ChE) and polyoxyethylene-p-t-octyl phenyl ether (Triton-X100) which is a surfactant having the action of activating the reaction of cholesterol esterase in water, and the mixed solution was dropped in an amount of 0.4 µl on a surface of the cover member consisting of the laminate of the spacer 7 and the cover 10 exposed to the sample solution supply pathway and was freeze-dried to form the enzyme layer 13. The cover member was bonded to the base plate 1 in the positional relationship as shown by the dash-dotted lines in FIG. 1 to produce a biosensor. The air vent had the shape as illustrated in FIG. 3. The round part of the air vent is constituted of a circle having a diameter of 2.0 mm.

The hemocyte filter 12 was disposed at a position contacting the opening 8a of the sample solution supply pathway. The hemocyte filter is a Whatman F147-11 (370 µm in thickness, produced by Whatman plc) which is cut into an isosceles triangle having a bottom length of 3 mm and a height of 5 mm, and this is inserted 1 mm from the vertex into the sample solution supply pathway 9. In order to prevent blood from directly reaching the opening 8a of the sample solution supply pathway 9 along the outer periphery of the hemocyte filter, the hole 6 is formed in a part of the base plate 1 at which the hemocyte filter is placed. Incidentally, the sample solution supply pathway has a tunnel-like structure which is 3.5 mm in length, 0.8 mm in width and 0.1 mm in thickness. The thickness of 0.1 mm is the thickness of the spacer 7.

The biosensor thus produced was supplied with 10 µl of blood as the sample solution from the primary side of the hemocyte filter 6, and three minutes later, a pulse voltage of +0.5 V was applied onto the measuring electrode in the anodic direction with respect to the counter electrode, and 5 seconds later, the current value was measured. The air vent had the shape as illustrated in FIG. 3: the ceiling 9b of the sample solution supply pathway of the cover 10 had a part facing the air vent 11, and this part protruded at its center toward the air vent 11 relative to the side walls 9a of the sample solution supply pathway. Thus, at the interface between the enzyme layer 13 and the air vent, the enzyme layer 13 descended in a gradual slope to the cover 10. It was therefore confirmed that the liquid filtered by the hemocyte filter filled the sample solution supply pathway within 1 minute after the supply of blood, completely dissolving the enzyme layer 13. The measurements of the response exhibited response characteristics dependent on the total cholesterol concentration.

### Comparative Example 1

A biosensor having the same structure as the biosensor of Example 1 except that the air vent had a round shape as in FIG. 10 was produced. Thus, the enzyme layer 53 carried on the cover member had the shape as illustrated in FIG. 11, wherein the enzyme layer 53 had an increased thickness and a distorted surface shape near the interface between itself and the air vent. In this biosensor, upon the supply of blood as the sample solution, some part of the enzyme layer remained undissolved when the liquid filtered by the hemocyte filter permeated the sample solution supply pathway to dissolve the enzyme layer.

### Example 2

The biosensor of this example has almost the same structure as Example 1, but is different in that the air vent has the shape as illustrated in FIG. 7. The width L of the opening of the air vent is 0.5 mm. In the case of the biosensor of Example 1, when the viscosity of the sample solution was low, for example, when the sample solution was standard serum, the sample solution overflowed into the air vent in some cases. Although the same tendency was also observed in this example, the amount of the overflowed sample solution was less than that of the biosensor of Example 1, because the area of the opening was smaller than that of Example 1.

### Example 3

The biosensor of this example has the structure as illustrated in FIG. 9.

In the same manner as in Example 1, the CMC layer 34 was formed over the electrode system on the base plate 21. Subsequently, an aqueous solution containing 250 U/ml glucose oxidase and 50 millimole/l potassium ferricyanide was dropped in an amount of 0.4 µl over the CMC layer 34 and was dried at 50°C for 10 minutes to form the layer 35 containing the enzyme and potassium ferricyanide. Further, a 0.5 wt% solution of lecithin in butanol was dropped in an amount of 3 µl and was dried to form the lecithin layer 36. The lecithin layer is just intended to improve the permeation of the sample solution and does not directly participate in the reaction. The air vent has the shape as illustrated in FIG. 7.

The biosensor thus produced was supplied with 3 µl of a glucose standard solution, which was prepared by dissolving glucose in a physiological saline solution, as the sample solution from the opening of the sample solution supply pathway, and one minute later, a pulse voltage of +0.5 V was applied onto the measuring electrode in the anodic direction with respect to the counter electrode, and 5 seconds later, the current value was measured. The measurements exhibited response characteristics dependent on the glucose concentration. In some of the sensors, the sample solution occasionally overflowed into the air vent, but since the area of the opening of the air vent was small as in FIG. 7, no influence on the response value was observed. The width L of the air vent in the longitudinal direction of the sample solution supply pathway was 0.5 mm. Incidentally, the sample solution supply pathway has a tunnel-like structure which is 4.5 mm in length, 2.0 mm in width and 0.313 mm in thickness. The thickness of 0.313 mm is the thickness of the spacer.

### Comparative Example 2

The biosensor has the same structure as Example 3 except that the air vent has a round shape as in FIG. 10, and exhibited response characteristics dependent on the glucose concentration in the same manner as in Example 3. However, in some of the sensors, the sample solution occasionally overflowed into the air vent. Since the area of the opening is larger than that of Example 3, the amount of overflowed sample solution is also larger.

### Industrial Applicability

As described above, the present invention can provide a biosensor capable of accurate measurements by making the amount of a sample solution introduced into a sample solution supply pathway constant. Also, it provides a biosensor having a structure which allows prompt and homogeneous dissolution of reagents necessary for measurements such as an enzyme and an electron mediator into the sample solution, thereby making prompt and accurate measurements possible.

## Claims

1. A biosensor comprising: an electrically insulating base plate; an electrode system comprising at least a measuring electrode and a counter electrode formed on said base plate; a cover member which is joined to said base plate to form a sample solution supply pathway for supplying a sample solution to said electrode system between the cover member and the base plate; and a reaction reagent carried at a position which is exposed to said sample solution supply pathway,
wherein said cover member comprises a cover which is disposed at a position opposite to the electrode system formed on said base plate and a spacer which is inserted between said cover and said base plate, said spacer having a slit for forming said sample solution supply pathway,
said cover has an air vent communicating with the slit of said spacer, and
a part of said cover facing the air vent and constituting the ceiling of the sample solution supply pathway protrudes at its center toward the air vent relative to the side walls of the sample solution supply pathway.

2. The biosensor in accordance with claim 1, wherein the width of the opening of said air vent in the longitudinal direction of said sample solution supply pathway is 0.5 mm at the maximum and 0.1 mm at the minimum.

3. The biosensor in accordance with claim 1 or 2, wherein at least a part of the reaction reagent is carried on a surface of said cover member exposed to the sample solution supply pathway.

4. The biosensor in accordance with one of claims 1 to 3, wherein the reaction reagent comprises at least cholesterol oxidase and an enzyme capable of hydrolyzing cholesterol ester.
